# EUROPEAN PATENT SPECIFICATION

(11) **EP 1 469 868 B1**
(45) Date of publication and mention of the grant of the patent: **17.01.2007**
(21) Application number: 02801970.1
(22) Date of filing: 30.08.2002
(51) Int. Cl.: A61K 36/00, A61P 35/00

(54) **KURKUMIN COMPOSITION FOR THE CURE OF LARGE INTESTINE POLYPS**
KURKUMIN-ZUSAMMENSETZUNG ZUR HEILUNG VON DICKDARMPOLYPEN
COMPOSITION A BASE DE CURCUMIN DESTINEE A SOIGNER LES POLYPES DU GROS INTESTIN

(30) Priority: 25.10.2001 HR 20010790
(43) Date of publication of application: 27.10.2004
(73) Proprietor: Leko, Vladimir, 34000 Slavonska Pozega (HR)
(72) Inventor: Leko, Vladimir, 34000 Slavonska Pozega (HR)
(74) Representative: von Füner, Nicolai
(86) International application number: PCT/HR2002/000039
(87) International publication number: WO 2003/035088

(56) References cited:
- DATABASE BIOSIS [Online] BIOSCIENCES INFORMATION SERVICE, PHILADELPHIA, PA, US; May 2001 (2001-05) COLLETT GAVIN PATRICK ET AL: "Curcumin modifies Apcmin apoptosis resistance and inhibits 2-amino 1-methyl-6-phenylimidazo(4,5-b)pyridine (PhIP) induced tumour formation in Apcmin mice." Database accession no. PREV200100285950 XP002225774 & CARCINOGENESIS (OXFORD), vol. 22, no. 5, May 2001 (2001-05), pages 821-825, ISSN: 0143-3334
- DATABASE BIOSIS [Online] BIOSCIENCES INFORMATION SERVICE, PHILADELPHIA, PA, US; November 2000 (2000-11) SKRZYPCZAK-JANKUN EWA ET AL: "Curcumin inhibits lipoxygenase by binding to its central cavity: Theoretical and X-ray evidence." Database accession no. PREV200000521828 XP002225775 & INTERNATIONAL JOURNAL OF MOLECULAR MEDICINE, vol. 6, no. 5, November 2000 (2000-11), pages 521-526, ISSN: 1107-3756
- DATABASE BIOSIS [Online] BIOSCIENCES INFORMATION SERVICE, PHILADELPHIA, PA, US; January 2002 (2002-01) MIQUEL J ET AL: "The curcuma antioxidants: Pharmacological effects and prospects for future clinical use. A review." Database accession no. PREV200200148969 XP002225776 & ARCHIVES OF GERONTOLOGY AND GERIATRICS, vol. 34, no. 1, January 2002 (2002-01), pages 37-46, ISSN: 0167-4943

## Description

### Field which the invention refers to

This invention refers to the phytopharmacy area, and more closely to the pharmaceutical formulation which assists the performance of polyp remedies.

### Technical problem

The most common benign tumours are fibroms, lipoms, neuromas, adenomyoses and polypoms. Simultaneous emergence of numerous polyps inside one organ is called polypasis. The most commonly, polyps emerge in the nose and uterus, and the digestive system polyps often alter:
a) the percentage of cancerous alteration of the stomach polyp: 18-28%
b) large intestine polyps alter 30-45%
c) familial polypasis: 65%
d) chronic ulcerous colitis: 10-15%

Technical problem, to which the solution is offered in this application, is to:
1. inhibit the COX-2 activity
2. without affecting the COX-1 in the process.

This means that a selective inhibitor is sought, the one that must be:
- non-damaging (to avoid side effects of heretofore known non-selective or not sufficiently selective inhibitors);
- and that it cannot overdose.

### State of the art

In case of the all already mentioned intestine and stomach inflammations, and in case of cancer, large doses of the enzyme-CYCLOOXIGENASE-2 (COX-2) have been spotted. If the development of COX-2 is prevented by selective inhibitor, the chances for the rehabilitation of inflammations are high, as well as the chances for the decrease of polyps and the prevention of their alteration into cancerous substances. Such inhibitor is to be used by persons suffering from all forms of polypases, as prevention against turning into cancer. In order to prevent the development of the COX-2, and to avoid the destruction of the COX-1 (the enzyme which plays a role in the production of prostaglandins that protect mucous membrane of the stomach), a lot of selective inhibitors have been produced: aspirin, voltaren, celebex, ibuprofen, etc.

However, certain side effects have been noticed in case of most of the known inhibitors. Especially the bleeding of digestive system and the creation of stomach ulcer have been problematic. This side effects occur because the inflammation is reduced when the enzyme COX-1 is being blocked, but the mucous membrane in the stomach is also being destructed in the process.

Some work on curry powder had been carried out, especially in Great Britain, but it was determined that large amount of it was needed. In case of "Kurkumin" this problem is solved with the long-term use of our preparation, thus providing good results.

### Description of the invention in detail

The herb Curcumae Longe Rhizomae - rhizome, originating from India, is consisted of:
a) p-tolimethyl carbinol (C₈H₁₀O)
b) ether oils
c) bitter substances
d) turmeric (yellow)

Rhizome root is grinded into powder, the size of 0,01 - 10 µm, and grapefruit (Citrus Maxima bark, grinded to the same size, is added to it. The percentage share of the grapefruit bark is 5 - 10%, preferably 5 w/w %. The grapefruit bark contains ascorbic acid (C vitamin), and other acids originating from fruit are well presented. It is known that turmeric substances are joined with C vitamin, sometimes causing light hyper-vitaminosis. Besides, grapefruit bark disguises very unpleasent rhizome root flavour. The gelatinous capsules are filled with this mixture. P-tolyl carbinol, as the main active agent, produces an effect on cyclooxigenase-2 enzyme (COX-2) - C₂₀H₃₂O₂. High concentration of COX-2 was spotted in case of some inflammatory processes (polypasis, arthritis, tumours). Under the effect of p-tolyl carbinol, the active COX-2 turns into inactive COX-2 - C₂₀H₃₃O₃. What remains is p-dimethyl benzen (C₈H₁₀) which is extracted from the organism through urine. Inflammations are decreased simultaneously and without iritating the stomach.

Preparation of the composition:
1. It slows the development of polyps in the large intestine by selective production of effects on the cyclooxigenase enzyme (COX-2). In case of large intestine polypasis, the symptoms of the state the organisam is in, and which are visible to human eye, improve as soon as ten days after the first administration, with the dose of three "Kurkumin" capsules. Each capsule contains 100-500 mg, preferably 200 of rhizome herb root. Persons who had been administering our preparation gave up the surgery and are able to live normally, without problems caused by polyps. The most important fact is that polyps do not turn into cancerous substances. However, the cure is long-term and "Kurkumin" is to be administered for months in order to prevent the return of polypasis symptoms.
2. It improves the creation and secretion of gall;
3. Successfully effects the inflammation of knuckles;
4. It effects haemorrhoids. They dry like polyps, meaning that COX-2 well blocked.

### SIDE EFFECTS

Due to the lack of K vitamin, the composition is not to be administered by persons with disordered coagulation of blood. It should neither be used by children, persons over 65, and women who wish to get pregnant.

## Claims

1. Pharmaceutical composition **characterized by** the fact that it consist of following dried and grinded ingredients:
| | |
|---|---|
| Curcumae longae rhizomae | 90-95 w/w % |
| Citrus Maxima | 5-10 w/w % |

2. Pharmaceutical composition according to claims 1, **characterized by the fact** that it consist, of following dried and grinded ingredients:
| | |
|---|---|
| Curcumae longae rhizomae | 95 w/w % |
| Citrus Maxima | 5 w/w % |

3. Pharmaceutical composition according to the claims 1-2, **characterized by** the fact that it is prepared in form of capsules.

4. Pharmaceutical composition according to claim 3, **characterized by the fact** that the capsule contains 100-500 mg of rhizome herb root.

5. Pharmaceutical composition according to any of claims 1-4, **characterized by the fact** that the root of Curcumae longae rhizomae herb is grinded to the size of 0,01-10 µm.

6. Pharmaceutical composition according to claims 1 - 2, **characterized by the fact** that, by functioning as selective inhibitor on cyclooxigenase-2 enzyme, it prevents the growth of large intestine polyps, and their turning into cancerous substances.

7. Use of the composition according to claims 1, 2. 4 and 5, **characterized by the fact** that it is used for the manufacture of a medicament for the prevention of the growth of large intestine polyps, and their alteration into cancerous substances.

## Patentansprüche

1. Pharmazeutische Zubereitung, **dadurch gekennzeichnet, dass** sie aus folgenden getrockneten und gemahlenen Komponenten besteht:
| | |
|---|---|
| Curcumae longae rhizomae | 90-95 Gew./Gew.-% |
| Citrus Maxima | 5-10 Gew./Gew.-%. |

2. Pharmazeutische Zubereitung nach Anspruch 1, **dadurch gekennzeichnet, dass** sie aus folgenden getrockneten und gemahlenen Komponenten besteht:
| | |
|---|---|
| Curcumae longae rhizomae | 95 Gew./Gew.-% |
| Citrus Maxima | 5 Gew./Gew.-%. |

3. Pharmazeutische Zubereitung nach einem der Ansprüche 1-2, **dadurch gekennzeichnet, dass** sie in Form von Kapseln hergestellt ist.

4. Pharmazeutische Zubereitung nach Anspruch 3, **dadurch gekennzeichnet, dass** die Kapsel 100-500 mg Rhizom enthält.

5. Pharmazeutische Zubereitung nach einem der Ansprüche 1-4, **dadurch gekennzeichnet, dass** die Curcumae longae rhizomae bis zu einer Größe von 0,01-10 µm vermahlen ist.

6. Pharmazeutische Zubereitung nach Anspruch 1-2, **dadurch gekennzeichnet, dass** sie in ihrer Funktion als selektiver Inhibitor auf das Enzym Cyclooxigenase-2 das Wachstum großer Darmpolypen und deren Veränderung zu kanzerogenen Substanzen verhindert.

7. Verwendung der Zubereitung nach den Ansprüchen 1, 2, 4 und 5 zur Herstellung eines Medikaments zur Verhinderung des Wachstums großer Darmpolypen und deren Veränderung zu kanzerogenen Substanzen.

## Revendications

1. Composition pharmaceutique **caractérisée par le fait qu'**elle est constituée par les ingrédients secs et broyés suivants :
| | |
|---|---|
| Curcumae longae rhizomae | 90 à 95 % en poids (w/w %) |
| Citrus Maxima | 5 à 10 % en poids (w/w %) |

2. Composition pharmaceutique selon la revendication 1, **caractérisée par le fait qu'**elle est constituée par les ingrédients secs et broyés suivants :
| | |
|---|---|
| Curcumae longae rhizomae | 95 % en poids (w/w %) |
| Citrus Maxima | 5 % en poids (w/w %) |

3. Composition pharmaceutique selon les revendications 1 à 2, **caractérisée par le fait qu'**elle est préparée sous la forme de gélules.

4. Composition pharmaceutique selon la revendication 3, **caractérisée par le fait que** la gélule contient 100 à 500 mg de racine de plante à rhizomes.

5. Composition pharmaceutique selon les revendications 1 à 4, **caractérisée par le fait que** la racine de la plante Curcumae longae rhizomae est broyée jusqu'à la dimension de 0,01 à 10 µm.

6. Composition pharmaceutique selon les revendications 1 à 2, **caractérisée par le fait que**, en fonctionnant comme un inhibiteur sélectif sur l'enzyme cyclo-oxygénase 2, elle empêche la croissance de polypes dans le gros intestin, et leur transformation en substances cancéreuses.

7. Utilisation de la composition selon les revendications 1, 2, 4 et 5, **caractérisée par le fait qu'**elle est utilisée pour la fabrication d'un médicament destiné à la prévention de la croissance de polypes dans le gros intestin et de leur modification en substances cancéreuses.
